(19) Europäisches Patentamt
European Patent Office
Office européen des brevets

(11) **EP 2 934 672 B1**

(12) **EUROPÄISCHE PATENTSCHRIFT**

(45) Veröffentlichungstag und Bekanntmachung des Hinweises auf die Patenterteilung:
**26.09.2018 Patentblatt 2018/39**

(21) Anmeldenummer: **13818183.9**

(22) Anmeldetag: **21.10.2013**

(51) Int Cl.:
**A61N 1/40** (2006.01)

(86) Internationale Anmeldenummer:
**PCT/EP2013/003158**

(87) Internationale Veröffentlichungsnummer:
**WO 2014/094930 (26.06.2014 Gazette 2014/26)**

(54) **VORRICHTUNG ZUR BEHANDLUNG VON BIOLOGISCHEM GEWEBE MIT EINEM NIEDERDRUCKPLASMA**

DEVICE FOR TREATING BIOLOGICAL TISSUE WITH A LOW-PRESSURE PLASMA

DISPOSITIF DE TRAITEMENT DE TISSU BIOLOGIQUE AU MOYEN D'UN PLASMA À BASSE PRESSION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.12.2012 DE 102012025080**

(43) Veröffentlichungstag der Anmeldung:
**28.10.2015 Patentblatt 2015/44**

(73) Patentinhaber: **NorthCo Ventures GmbH & Co. KG 90491 Nürnberg (DE)**

(72) Erfinder:
• **SRB, Josef 38801 Blatna (CZ)**
• **KOROUS, Josef 38801 Blatna (CZ)**
• **HINTERKOPF, Jan 52070 Aachen (DE)**

(74) Vertreter: **Preusche, Rainer et al Preusche & Partner Patent- und Rechtsanwälte mbB Schlossstraße 1 56068 Koblenz (DE)**

(56) Entgegenhaltungen:
**EP-A1- 2 394 693        WO-A2-2006/116252 DE-U1-202005 004 364**

## Beschreibung

Die Erfindung betrifft eine Vorrichtung zur Behandlung von biologischem Gewebe mit einem Niederdruckplasma nach dem Oberbegriff des Patentanspruchs 1.

Dass Plasmen antimikrobielle Eigenschaften besitzen, ist bekannt. Die Ursachen der antibakteriellen Wirkung eines Plasmas liegen in Hitze, Austrocknung, Scherspannung, UV-Strahlung, freie Radikale und Ladungen. Bei Niederdruckplasmen, die auch kalte Plasmen genannt werden, spielt die Hitze eine untergeordnete Rolle, da diese Plasmen bei Raumtemperatur betrieben werden. In solchen Niederdruckplasmen entstehen besonders reaktive Partikel, wie beispielsweise verschiedene Sauerstoff- oder Stickstoffspezies, die eine ausreichend hohe Lebensdauer aufweisen, um bei einer indirekten Exposition organische Verbindungen zu schädigen. Zu diesen Partikeln zählen unter anderem atomarer Sauerstoff, Superoxidradikale, Ozon, Hydroxylradikale, Stickstoffmonooxid und Stickstoffdioxid. Diese Partikel zeigen eine zerstörerische Wirkung auf unterschiedlichste Zellkomponenten.

Werden Zellwände von Bakterien, Keimen, Viren, Pilzen oder anderen vergleichbaren Mikroorganismen dem Plasma direkt ausgesetzt, so laden sich diese aufgrund des Beschusses mit den im Plasma vorhandenen Elektronen negativ auf. Aufgrund der elektrostatischen Abstoßung führt dies zu mechanischen Spannungen bis hin zur Überschreitung der Zugfestigkeit und der Zerstörung der Zellwand. Aber nicht nur mechanische Verspannungen aufgrund der Ladung können die Zellwände zerstören, sondern auch die Störung des Ladungsgleichgewichts der Zellwand durch verschiedene, weitere elektrostatische Wechselwirkungen und der Elektrolyse, z. B. durch Änderung der Permeabilität der Zellwände. Ein Mechanismus zur Inaktivierung von Mikroorganismen ergibt sich auch aus den sehr energiereichen Ionen, die in kapazitiv gekoppelten Systemen weit über 100 eV aufweisen können. Ein Beschuss mit solchen Spezies kann die strukturelle Integrität der Zellen verändern bzw. sie zerstören; allerdings ist eine Vorrichtung zur Erzeugung solcher Ionenstrahlen aufwendig und nur mit sehr hohem apparativen Aufwand zur Behandlung von lebendem biologischen Gewebe, insbesondere menschlichen oder tierischen Geweben, geeignet.

Niederdruckplasmen sind daher besonders gut zur Behandlung von Gewebe von Menschen oder Tieren, insbesondere von Hautoberflächen, offenen Wunden, des Zahnfleischs, der Mundhöhle oder dergleichen geeignet, um eine Desinfektion des Gewebes, insbesondere die Abtötung von Bakterien, Keimen, Viren, Pilzen oder anderer vergleichbarer Mikroorganismen, die in oder auf dem Gewebe platziert sind, zu erreichen.

Aus der DE 10 2005 000 950 B3 ist eine Vorrichtung und ein Verfahren zur Behandlung von biologischem Gewebe mit Ozon bekannt. Diese Vorrichtung besteht im Wesentlichen aus einem mittels einer Steuereinrichtung in Spannung und/oder Stromstärke regelbaren Transformator zur Erzeugung spezieller gerichteter Spannungs- oder Stromimpulse verschiedenster Charakteristik mit oder ohne Gleichspannungsanteil. Der Gleichspannungsanteil wird dabei durch zusätzliche Elektroden am zu behandelnden biologischen Gewebe mithilfe einer externen Spannungsquelle oder Schaltung aufgebaut. Die Primärspule des Transformators ist die von hochfrequentem Wechselstrom durchflossene Spule eines gedämpften Schwingungskreises. Die Sekundärspule bildet zusammen mit dem aufzuladenden Kondensator einen Schwingkreis, dessen Frequenz mit der des Transformators übereinstimmt. Als Stromquelle dient oftmals ein Resonanztransformator. Damit ist die Behandlung von verschiedenen Gewebetypen, wie beispielsweise Mundschleimhäute für Behandlungen in der Mundhöhle oder Hautgewebe für dermatologische Behandlungen mit Ozon möglich. Die Leistungseinstellung des Pulsgenerators erfolgt über Bedienelemente, die an der Steuereinrichtung angeordnet sind. Mit einem Drehknopf erfolgt die Einstellung der Leistung innerhalb eines Leistungsbereichs durch den Anwender beziehungsweise den behandelnden Arzt anhand einer Zahlenskala.

Allerdings ist bei dieser bekannten Vorrichtung eine möglich, dass in Abhängigkeit der konkreten Behandlungssituation zu einer unterschiedlichen Leistungs- beziehungsweise Stromaufnahme des zu behandelnden Gewebes kommt, wodurch einerseits die Funktion der Vorrichtung negativ beeinflusst werden kann. Andererseits kann durch eine erhöhte Leistungsbeziehungsweise Stromaufnahme auch der zu behandelnde Patient geschädigt werden.

Das Dokument WO-A-2006/116252 offenbart den nächstliegenden Stand der Technik.

Es ist daher zum einen Aufgabe der Erfindung, eine Vorrichtung und ein zur Behandlung von biologischem Gewebe mit einem Niederdruckplasma mit den Merkmalen des Oberbegriffs des Patentanspruchs 1 derart weiterzubilden, dass bei einer veränderten, insbesondere bei einer erhöhten Leistungs- beziehungsweise Stromabgabe durch die Vorrichtung die Stromversorgung unterbricht. Zum anderen ist es Aufgabe der Erfindung, eine Informationseinrichtung für eine solche Vorrichtung zur Verfügung zu stellen, die dem Anwende darüber den Abstand zwischen Sonde und zu behandelnden Gewebe sowie die Behandlungszeit informiert.

Vorrichtungsmäßig wird diese Aufgabe durch eine Vorrichtung mit allen Merkmalen des Patentanspruchs 1 gelöst.

Die erfindungsgemäße Vorrichtung zur Behandlung von biologischem Gewebe mit einem Niederdruckplasma enthält im Wesentlichen

einen Transformator zur Erzeugung eines hochfrequenten elektromagnetischen Feldes, eine Sonde, welche mit dem Transformator elektrisch koppelbar ist und einer Steuerungseinrichtung zur Steuerung des durch den Transformator erzeugten hochfrequenten elektromagnetischen Feldes, wobei

der Sonde eine Informationseinrichtung zugeordnet ist, mit welcher

die Zeitdauer der Leistungsabgabe der Vorrichtung an das Gewebe messbar sowie optisch und/oder akustisch anzeigbar ist und/oder

die Stromstärke beziehungsweise die Leistung, welche durch die Vorrichtung an das Gewebe abgegeben wird, messbar ist und beim Überschreiten eines vordefinierten Grenzwertes die Energieversorgung der Vorrichtung unterbricht und/oder der Abstand einer Behandlungsfläche der Sonde zu dem zu behandelnden Gewebe messbar sowie optisch und/oder akustisch anzeigbar ist.

Durch diese spezielle Ausgestaltung der erfindungsgemäßen Vorrichtung ist es zum einen erreicht, dass trotz eine erhöhte Leistungs- beziehungsweise Stromabgabe in das zu behandelnde Gewebe sofort unterbrochen wird, so dass eine Schädigung der erfindungsgemäßen Vorrichtung und des zu behandelnden Patienten durch eine zu hohen Strom vermieden wird. Zum anderen wird dem Anwender die Behandlungsdauer optisch und/oder akustisch dargestellt, wozu die Vorrichtung entsprechende optische Anzeigeeinrichtungen und Lautsprecher aufweist. Weiterhin können diese optische Anzeigeeinrichtungen und Lautsprecher dazu genutzt werden, dem Anwender über den Abstand der Behandlungsfläche der Sonde zu dem zu behandelnden Gewebe zu informieren. Bewegt sich der Abstand aus einem zur Behandlung optimalen Abstand, der vom Fachmann festgelegt werden kann, heraus, wird dies dem Anwender auf einer optischen Anzeigeeinrichtung angezeigt beziehungsweise durch einen Lautsprecher akustisch signalisiert, wobei anhand eines akustischen Warnsignals auch festgestellt werden kann, ob der Abstand zu gering oder zu groß ist.

Vorteilhafterweise umfasst der Transformator ein Transformatorgehäuse, welches eine der Kupplung für die Sonde gegenüberliegende Kupplung zum elektrischen/elektronischem Anschluss der Steuereinrichtung aufweist, wobei das Transformatorgehäuse vorzugsweise als Handgriff ausgebildet und entsprechend ergonomisch geformt ist. Diese Maßnahme zielt auf eine kompakte Bauweise der gesamten erfindungsgemäßen Vorrichtung, da sowohl der Transformator selbst als auch die Steuerungseinheit innerhalb des Transformatorgehäuses anordnenbar ist. Lediglich die Sonde zur Behandlung des biologischen Gewebes und gegebenenfalls eine externe Energiequelle zur Energieversorgung der erfindungsgemäßen Vorrichtung sind nicht innerhalb des Transformatorgehäuses angeordnet. Die ergonomische Ausgestaltung des Transformatorgehäuses als Handgriff, der in seiner Grundform zylindrisch ausgebildet ist, erlaubt ferner eine angenehme und sichere Handhabung der erfindungsgemäßen Vorrichtung durch den Anwender.

Aus den gerade genannten Gründen der kompakten Bauweise und der einfachen, sicheren und angenehmen Handhabung der erfindungsgemäßen Vorrichtung ist daher nach einem vorteilhaften Gedanken der Erfindung die Steuereinrichtung in dem Transformatorgehäuse angeordnet.

Allerdings kann es für bestimmte Anwendungen sinnvoll sein, die Steuereinrichtung außerhalb des Transformatorgehäuses anzuordnen. Insbesondere dann, wenn sehr filigrane Behandlungen durchgeführt werden müssen, bei denen zusätzliches Gewicht innerhalb des als Handgriff ausgebildeten Transformatorgehäuses hinderlich bei der Handhabung der erfindungsgemäßen Vorrichtung ist.

Die Steuereinrichtung ist an eine elektrische Energiequelle anschließbar, damit die erfindungsgemäße Vorrichtung mit der für den Betrieb notwendigen elektrischen Energie versorgt werden kann. Dabei ist insbesondere bei einer innerhalb des als Handgriff ausgebildeten Transformatorgehäuses angeordneten Steuereinrichtung eine Energiequelle in Form von Batterien oder Akkumulatoren, die ebenfalls im Transformatorgehäuse untergebracht, aber auch außerhalb des Transformatorgehäuses angeordnet sein können. Dies ist insbesondere deshalb sinnvoll, da die gesamte erfindungsgemäße Vorrichtung dann unabhängig von einer stationären Energiequelle und insbesondere unabhängig von einem öffentlichen beziehungsweise nichtöffentlichen elektrischen Netz betrieben werden kann. Allerdings ist es natürlich auch denkbar, als Energiequelle eine stationäre Energiequelle oder ein öffentliches beziehungsweise nichtöffentliches elektrischen Netz vorzusehen, mit welcher die Steuereinheit verbindbar ist.

Die Sonde mit der die eigentliche Behandlung durchgeführt wird, da mit ihr das erforderliche Niederfrequenzplasma zur Anwendung auf dem zu behandelnden Gewebe erzeugt wird, ist vorzugsweise als Glassonde ausgebildet ist. Solche Glassonden sind einfach zu handhaben und in der Anwendung an oder in biologischem Gewebe physiologisch unbedenklich.

Dabei hat es sich bewährt, die Glassonde unter Unterdruck, vorzugsweise unter Unterdruck von 500 Pa bis maximal 3000 Pa, mit einem leitenden Gas, vorzugsweise mit einem Edelgas oder Edelgasgemisch zu füllen. Mit solchen leitenden Gasen, insbesondere Edelgasen und Edelgasgemischen, vorzugsweise aus Argon und/oder Neon, ist die Herstellung von Niederfrequenzplasmen und somit die gesamte erfindungsgemäße Vorrichtung besonders effizient. Die Glassonde ist an ihrem einen Ende durch einen Metallkontakt geschlossen, durch welchen die durch den Transformator gelieferte hochfrequente Hochspannung in das Innere der Glassonde geführt wird. Innerhalb der Glassonde wird das Gas dem hochfrequenten elektromagnetischen Feld ausgesetzt und somit eine Glimmentladung erzeugt. Die Leistung des Transformators ist dabei durch die Steuereinrichtung derart regelbar, dass sich Spannungen im Bereich zwischen 1800 V und 35000 V einstellen lassen, sie über das leitfähige Gas innerhalb der Glassonde auf die Behandlungsfläche der Glassonde übertragen werden. Befindet sich die Behandlungsfläche der Glassonde unmittelbar über dem zu behandelnden biologischen Gewebe stellt sich diese Spannung dazwischen ein, gegebenenfalls in Abhängigkeit des elektrischen Widerstandes der Oberfläche des zu behandelnden biologischen Gewebes und dem Widerstand der Gase, insbesondere der Luft, zwischen Behandlungsfläche der Glassonde und Oberfläche des zu behandelnden biologischen Gewebes.

Damit die durch den Transformator zur Verfügung gestellte hochfrequente Hochspannung auch effizient durch die Sonde genutzt werden kann, ist eine gute und sichere elektrische Kontaktierung zwischen Transformator und Sonde unabdingbar. Dies wird nach einen eigenständigen Gedanken der Erfindung dadurch erreicht, dass die Sonde mittels einer Kontaktfeder elektrisch/elektronisch mit dem Transformator koppelbar ist. Dabei ist es zum einen denkbar, dass die Kontaktfeder an dem Transformator beziehungsweise dem Transformatorgehäuse angeordnet ist. Zum anderen kann die Kontaktfeder auch an der Sonde angeordnet sein. In beiden Fällen stellt die Kontaktfeder die elektrische Kontaktierung zwischen Sonde und Transformator sicher, auch wenn innerhalb der Kupplung zwischen Sonde und Transformator ein nicht erwünschtes Spiel auftritt.

Das Verfahren zur Behandlung von biologischem Gewebe mit einem Niederdruckplasma mit einer zuvor beschrieben Vorrichtung enthält im Wesentlichen folgende Verfahrensschritten:

a) Bereitstellen elektrischer Energie in Form von elektrischer Gleichspannung oder niederfrequenter Wechselspannung im Bereich von 12 V bis 600 V mit einer Stromstärke auf der Seite der Sekundärspule von 0,1 μA bis 300 μA,
b) Umwandlung der elektrischen Gleichspannung oder der elektrischen niederfrequenter Wechselspannung in hochfrequente Wechselspannung zwischen 10 kHz und 50 kHz,
c) Transformation der hochfrequenten Wechselspannung in einen Spannungsbereich zwischen 1800 V bis 35000 V,
d) Weiterleitung der hochfrequenten Wechselspannung in einen Spannungsbereich zwischen 1800 V bis 35000 V an eine Sonde (2), vorzugsweise eine Glassonde, welche über dem zu behandelnden biologischen Gewebe in einem Abstand zwischen 1 mm und 5 cm positioniert ist.

Hierbei sei darauf hingewiesen, dass bei Anwendungen im Dentalbereich, z. B. bei der Behandlungen der Mundschleimhäute in der Mundhöhle, die Stromstärke auf der Seite der Sekundärspule zwischen 0,1 μA und 100 μA gewählt wird, während bei Anwendungen auf sonstigen Gewebeoberflächen, insbesondere dermatologischen Behandlungen der übrigen Haut beziehungsweise des zu behandelten Patienten beziehungsweise gynäkologischen Anwendungen, die Stromstärke auf der Seite der Sekundärspule zwischen 0,1 μA und 300 μA gewählt wird.

Weitere Ziele, Vorteile, Merkmale und Anwendungsmöglichkeiten der vorliegenden Erfindung ergeben sich aus der nachfolgenden Beschreibung von Ausführungsbeispielen anhand der Zeichnungen. Dabei bilden alle beschriebenen und/oder bildlich dargestellten Merkmale für sich oder in beliebiger sinnvoller Kombination den Gegenstand der vorliegenden Erfindung, auch unabhängig von ihrer Zusammenfassung in den Ansprüchen oder deren Rückbeziehung.

Es zeigen:

Figur 1:  ein Transformators eines Ausführungsbeispiel einer erfindungsgemäßen Vorrichtung in einem Transformatorgehäuse,

Figur 2:  ein Transformatorgehäuse eines Ausführungsbeispiel einer erfindungsgemäßen Vorrichtung,

Figur 3:  eine Glassonde eines Ausführungsbeispiels einer erfindungsgemäßen Vorrichtung,

Figuren 4a-i sowie 4l-q:  verschiedene Ausführungsformen einer Sonde eines Ausführungsbeispiel einer erfindungsgemäßen Vorrichtung,

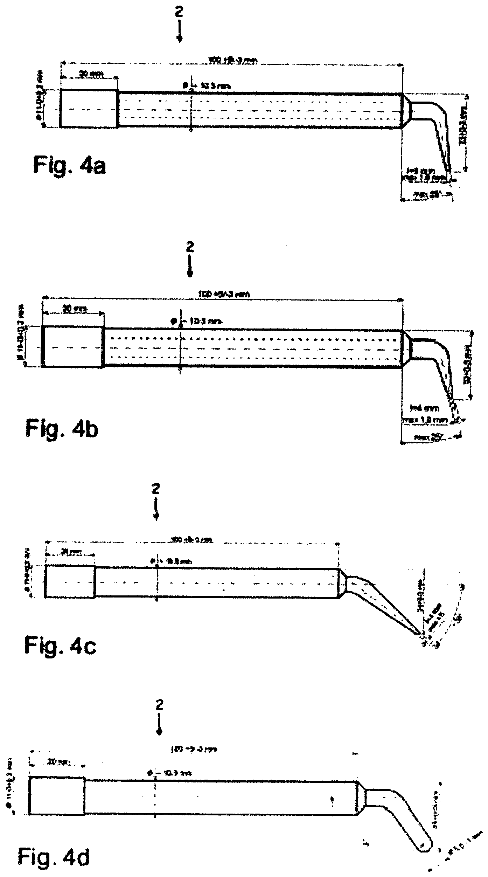

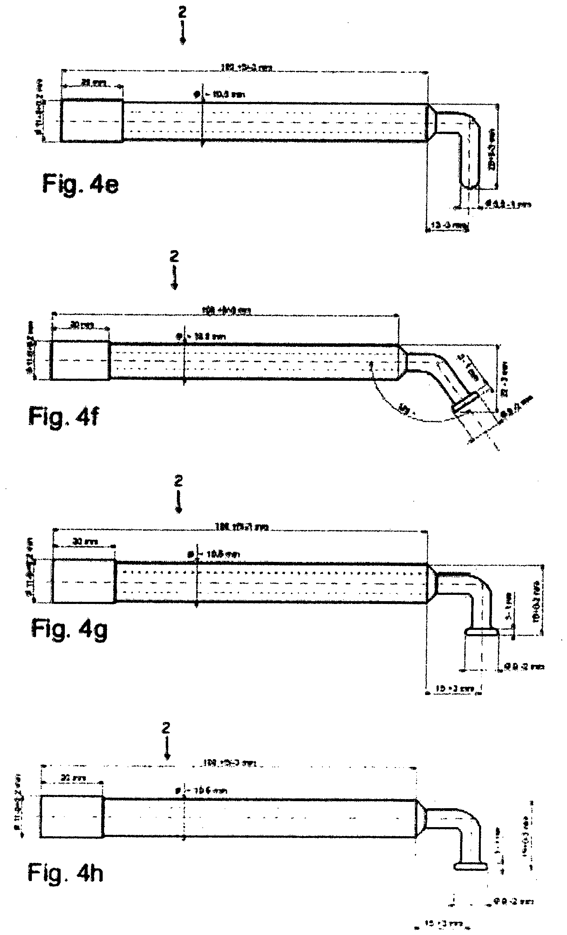

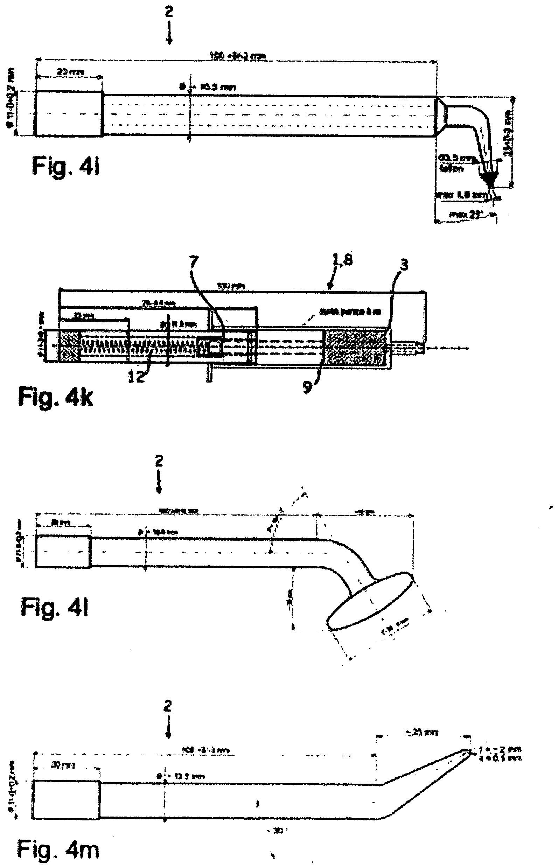

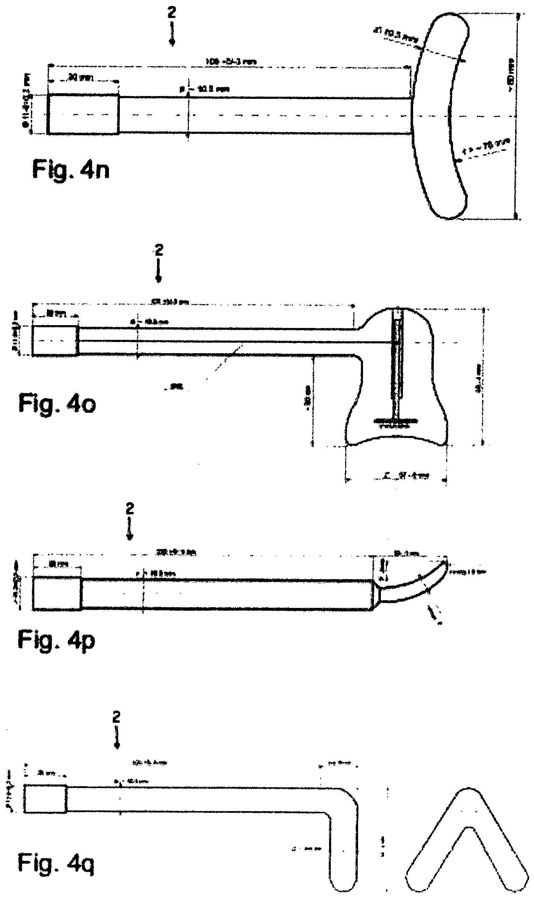

Figur 4 k:  eine Transformatorgehäuse mit Transformator und Steuereinrichtung eines Ausführungsbeispiel einer erfindungsgemäßen Vorrichtung zum Anschluss einer Sonde der Figuren 4a bis i und 4l bis q,

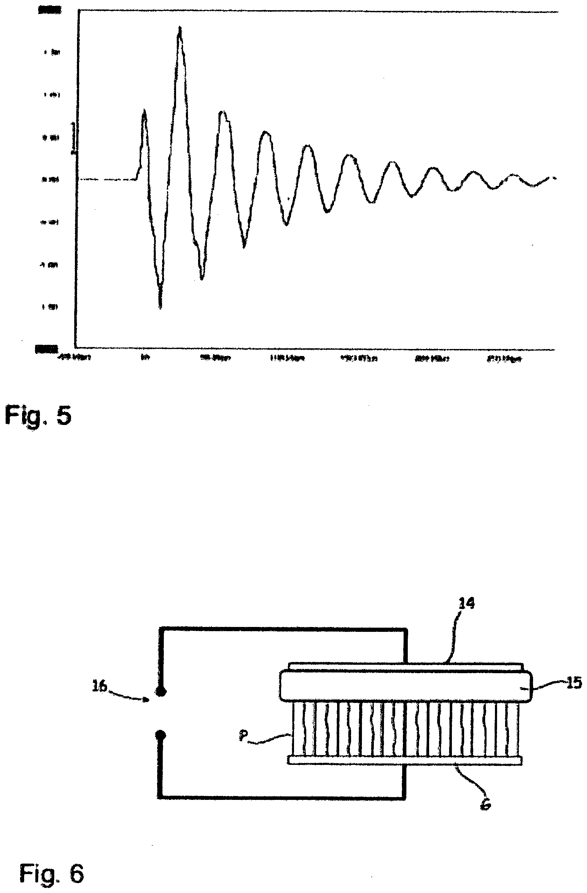

Figur 5:  typischer Pulsverlauf eines hochfrequenten Spannungspulses, bei dem die Stromstärke in μA gegen die Zeit aufgetragen ist und

Figur 6:  schematische Darstellung einer dielektrischen Barriereentladung.

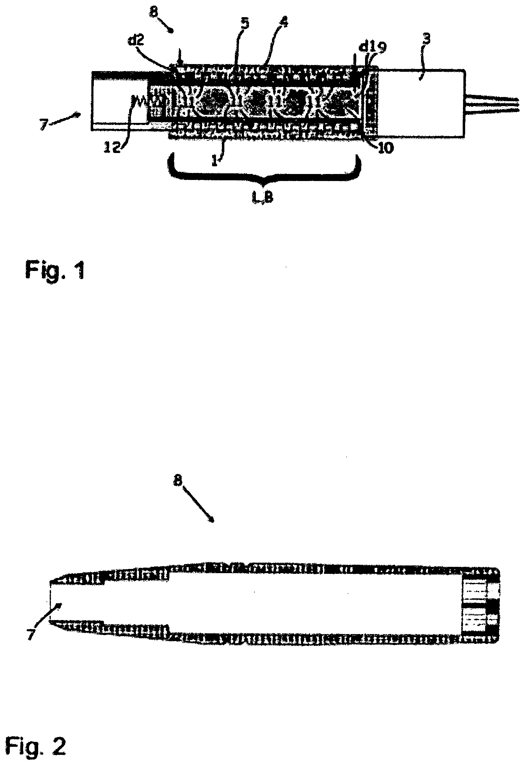

In den Figuren 1 bis 4q sind verschiedene Elemente von Ausführungsformen erfindungsgemäßer Vorrichtungen zur Behandlung von biologischem Gewebe mit einem Niederdruckplasma dargestellt, welche weiter unten näher erläutert werden.

Figur 1 zeigt beispielsweise eine Ausführungsform eines Transformatorgehäuses 8 einer erfindungsgemäßen Vorrichtung, in welches ein aus einer Primärspule 4 und einer Sekundärspule 5 gebildeter Transformator angeordnet ist, an den wiederum über eine Kupplung 9 eine Steuereinrichtung 3 angeschlossen ist. Die Steuereinrichtung 3 ist wiederum mit einer hier nicht dargestellten elektrischen Energiequelle 13 zur Einspeisung elektrischer Energie in den Transformator 1 verbunden. An dem der Kupplung 9 gegenüberliegenden Ende des Transformatorgehäuses 8 ist wiederum eine Kupplung 7 angeordnet, an welcher eine Sonde 2, vorzugsweise eine Glassonde angeordnet werden kann. Eine Kontaktfeder 12 stellt hierbei sicher, dass zwischen dem Transformator 1 und der Sonde 2 immer ein elektrischer Kontakt besteht. Das Transformatorgehäuse 8 ist vorliegend als ein Handgriff ausgebildet und erstreckt sich in seiner Längserstreckung in die gleiche Richtung wie die Primärspule 4 und die Sekundärspule 5.

Die Sekundärspule 5 ist in diesem Ausführungsbeispiel um einen Stabkern 10, der vorzugsweise aus einem Ferrit besteht, gewickelt, während die Primärspule 4 in einem Abstand um die Sekundärspule 5 gewickelt ist. Dieser Abstand nimmt dabei von dem der Kupplung 9 zugewandten Ende der Spule 4 und 5 von einem Abstand d1 bis zu dem der Kupplung 7 zugewandten Ende der Spulen 4 und 5 kontinuierlich bis zu einem Abstand d2 zu, so dass die Primärspule 4 konisch koaxial über der Sekundärspule angeordnet ist. In vorliegenden Ausführungsbeispiel weisen beide Spulen 4 und 5 die gleiche Länge L auf, so dass sie über ihre gesamte Länge einen Überlappungsbereich B bilden. Die Primärspule 4 nimmt dabei auch die Funktion eines elektromagnetischen Schirms wahr, beziehungsweise gewährleistet einen Abschirmeffekt, durch den elektromagnetische Störfelder nicht das durch den Transformator 1 erzeugte hochfrequente elektromagnetische Feld entscheidend stören können, so dass eine einwandfreie Funktion der erfindungsgemäßen Vorrichtung gegeben ist. Daneben können in einem Endabschnitt des Konverters noch Dichtungseinrichtungen vorgesehen sein.

Der als Hochspannungstransformator ausgebildete Transformator 1 ist in diesem Ausführungsbeispiel derart aufgebaut, dass die innere Sekundärspule 5 um einen Stabkern 10 aus Ferrit in Kammern 11 gewickelt ist. Bei der hier gezeigten Ausführungsform weist Sekundärspule 5 je 500 Windungen pro Kammer 11 auf; es sind jedoch auch andere Windungszahlen denkbar.

Der Transformator 1 nimmt einerseits die Aufgabe wahr, die von der Energiequelle 13 und der Steuereinheit 3 bereitgestellte hochfrequente Niederspannung in hochfrequente Hochspannung umzuwandeln. Andererseits nimmt er aber auch die Aufgabe wahr, die erzeugte Hochspannung, insbesondere über eine hier nicht dargestellte innere Glasröhre der als Glassonde ausgebildeten Sonde 2 an deren Behandlungsfläche zu leiten, die an dem Kupplung 7 gegenüberliegenden Ende der Sonde angeordnet ist..

Die Anordnung der Spulen 4 und 5 innerhalb des Transformators 1 führt zur Bereitstellung von Pulsen mit einer vorgegebenen Signalform, bevorzugt von sinusförmigen Pulsen und besonders bevorzugt von exponentiell gedämpften sinusförmigen Pulsen, wie sie beispielhaft in Figur 5 dargestellt sind und mit welchen ein kaltes Plasmas beziehungsweise ein Niederdruckplasma zwischen Behandlungsfläche der Sonde 2 und zu behandelnden Gewebe erzeugt werden kann.

Figur 2 zeigt den Aufbau eines Transformatorgehäuses 8 der Figur 2, welches aus einem elektrisch isolierendem Material, vorzugsweise einem Kunststoff hergestellt ist.

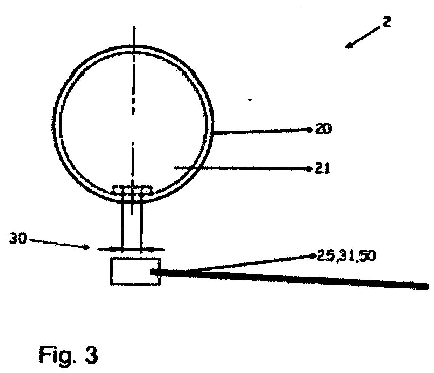

Figur 3 zeigt exemplarisch den Aufbau einer als Glassonde ausgebildeten Sonde 2 eines Ausführungsbeispiels einer erfindungsgemäßen Vorrichtung zusammen mit seinen elektrischen Komponenten zum Verbinden mit einem Transformator 1.

Die Sonde 2 der Figur 3 weist bevorzugt eine Steckeinrichtung 30 und bevorzugt weiterhin ein Verbindungskabel 31 mit einer vorgegebenen Länge auf. Weiterhin weist die Sonde 2 einen Sondenkörper 20 auf, der vorliegend als Hohlkugel mit einem Hohlraum 21 ausgebildet ist, aber auch als ein Körper mit einer anderen geometrischen Gestalt, etwa als Ellipsoid ausgeführt sein kann. Dabei kann dieser Sondenkörper 20 beispielsweise aus einem Metall, beispielsweise aus Edelstahl gefertigt sein. Vorteilhaft weist die Vorrichtung auch eine hier nicht dargestellte Halteeinrichtung zum Halten dieses Sondenkörpers 20 auf. Diese Haltevorrichtung kann dabei in eine Kupplung 7 des Transformatorgehäuses eingreifen, so dass die Sonde 2 sicher in beziehungsweise an dem als Handgriff ausgebildeten Transformatorgehäuse 8 gehalten ist. Vorteilhaft ist dieser Sondenkörper 20 aus einem stromleitenden Material hergestellt und weist bevorzugt zumindest abschnittsweise eine gekrümmte Oberfläche auf.

Weiterhin kann die Sonde 2 eine Verbindungseinrichtung 40 aufweisen, um den Sondenkörper an einem Träger, beispielsweise an einem stangenartigen Träger wie einem als Handgriff ausgebildeten Transformatorgehäuse 8 anzuordnen. Dabei ist es möglich, dass diese Verbindungseinrichtung 40 als Stopfen ausgeführt ist, der in das Innere des Sondenkörpers 20 eingebracht ist und der in den Träger beziehungsweise die Kupplung 7 des Transformatorgehäuses 8 eingeschoben wird.

Mittels einer elektrischen Verbindung 50 innerhalb der Verbindungseinrichtung 40 beziehungsweise der Halteeinrichtung ist die Sonde 2 durch eine Verbindungsleitung 25 mit einer Energiequelle 13 beziehungsweise der Steuerungseinrichtung 3 verbunden. Als Verbindungsleitung 2 kann beispielsweise ein zweiadriges Kabel verwendet werden.

Durch die Sonde 2 wird während der Behandlung der Stromkreis zwischen erfindungsgemäßer Vorrichtung und dem Patienten beziehungsweise des zu behandelnden Gewebes G im oder außerhalb des menschlichen oder tierischen Körpers geschlossen. Der erfindungsgemäße Vorrichtung erzeugt das kalte Plasma beziehungsweise das Niederdruckplasma.

Die Figuren 4a-i und 4l-q zeigen 15 verschiedene Beispiele als Glassonden ausgebildeter Sonden 2, deren

EP 2 934 672 B1

Behandlungsfläche je nach zu behandelndem biologischem Gewebe G schräg oder plan oder gebogen ausgeführt ist.

An dem die Kupplung 7 für die Sonde 2 aufweisenden Ende des Transformatorgehäuses 8 ist dieses mit einer Kontaktfeder 12 ausgestattet, welche elektronisch mit dem Transformator 1 verbunden ist. Wie bereits kurz erwähnt, stellt die Kontaktfeder 12 den Kontakt mit der Sonde 2 her. Durch den Kontakt werden die Spannungsimpulse auf die Sonde 2 übertragen. Die als Glassonde ausgebildete Sonde 2 ist in den Ausführungsbeispielen der Figuren 4a-i und 4l-q mit zwei Kammern ausgestattet. Die innere Kammer ist bevorzugt mit 100 % Neon gasgefüllt bei einem Unterdruck von 500 Pa bis 3000 Pa und leitet die Hochspannung an die Spitze der Instrumentensonde. Die äußere Kammer dient zur Isolierung und Schutz der inneren Kammer. Die innere Kammer besteht vorteilhaft aus Glas und die äußere Kammer kann aus dem Material Glas oder Edelmetall bestehen.

An dem der Behandlungsfläche gegenüberliegenden Ende ist die Sonde 2 mit einer Metallklappe geschlossen, welche zusammen mit der Kontaktfeder 12 und der Kupplung 7 die elektrische Steckverbindung mit dem in dem Transformatorgehäuse 8 angeordneten Transformator 1 herstellt.

Zwischen dem Behandlungsfläche der Sonde 2 und dem zu behandelnden biologischen Gewebe G bildet sich bei einem Abstand zwischen 1 mm und 5 mm durch die bereitgestellte hochfrequente Wechselspannung und dem typischen Pulsverlauf die Bildung des kalten Plasmas beziehungsweise des Niederdruckplasmas, mit welchem Bakterien, Keimen, Viren, Pilzen oder andere vergleichbare, dem Gewebe G anhaftenden Mikroorganismen abgetötet werden können.

Das Gas in der als Glassonde ausgebildeten Sonde 2 wird dabei dem erzeugten hochfrequenten, elektromagnetischen Wechselfeld ausgesetzt, um eine Glimmentladung (Mikroentladung) zu erzeugen. Die Leistung des Transformators 1 ist dabei über die Steuereinrichtung 3 so regelbar, dass sich Spannungen im Bereich zwischen 1,8 und 35 kV einstellen lassen, die über das leitfähige Gas auf die Behandlungsfläche der Sonde 2 übertragen werden. Befindet sich die Behandlungsfläche der Sonde 2 unmittelbar über dem zu behandelnden Gewebe G, stellt sich deren Spannung in Abhängigkeit von Hautwiderstand der Luft zwischen Instrumentensondenspitze und Hautoberfläche ein.

Das Verfahren zur direkten Erzeugung eines Niederdruckplasmas oder kalten Plasmas entspricht dem Aufbau der in Figur 6 dargestellten dielektrischen Barriereentladung. Die Anregungsspannung wird im Transformator 1 erzeugt. Die Sonde 2 bildet dabei eine Metallelektrode 14 und ein und Dielektrikum 15. Die Erdelektrode wird von dem zu behandelnden Gewebe G gebildet, so dass zwischen dem Gewebe G und der Metallelektrode 14 der Sonde 2 im Wesentlichen die durch den Transformator 1 gelieferte hochfrequente Anregungsspannung 16 anliegt. Das dargestellte Schema dient als Modell für die weiteren Betrachtungen.

Physikalische Betrachtung der Plasmabildung durch dielektrische Barriereentladung. Die dielektrische Barriereentladung, auch dielektrisch behinderte Entladung oder stille Entladung genannt, ruft bei Atmosphärendruck während der Zündphase nicht-thermische Plasmafilamente P hervor. Die dielektrisch behinderte Entladung oder stille Entladung ist in dieser Betrachtung neben der Koronaentladung eine Variante der Gasentladungen, die bei Atmosphärendruck während der Zündphase nicht-thermische Plasmafilamente P hervorruft. Der Unterschied zwischen beiden Gasentladungsformen liegt im Löschmechanismus der Entladungsfilamente. Im Fall der Koronaentladung ist er raumladungsorientiert und bei der Barriereentladung oberflächenladungsorientiert.

Der in Figur 6 dargestellte, grundsätzliche Aufbau besteht aus zwei Elektroden, einer Hochspannungselektrode 14 und einer Erdelektrode G, mit ein oder mehreren dielektrischen Barrieren 15 (Isolatoren) dazwischen. Zwischen Dielektrikum 15 und Erdelektrode G befindet sich ein Spalt, der in der Breite variabel ist, in der Größenordnung von einigen mm bis in den cm-Bereich. Die zu behandelnde Probe befindet sich auf der beziehungsweise bildet die Erdelektrode G. Um die Entladung zu erzeugen, wird eine Wechselspannung von 1-100 kV und Frequenzen von 10-50 kHz benötigt. Diese Entladung ist charakterisiert durch die Ausbildung von Mikroentladungen beziehungsweise Plasmafilamente P. Bei dieser Reaktion lagern sich Ladungsträger an die Oberfläche des Dielektrikums 15 an und schwächen das externe elektrische Feld, was zu einem Auslöschen der Plasmafilamente P führt. Das Dielektrikum 15 dient der Strombegrenzung und es ermöglicht, dass die Entladungen an einer Vielzahl statistisch gleichverteilter Punkte stattfinden können, womit eine flächige Plasmabehandlung der gesamten Oberfläche des zu behandelnden Gewebes G ermöglicht wird.

Die physikalische Betrachtung der Plasmabildung erfolgt nach der Methode von Paschen und Townsend. Die Analyse bezieht sich auf das in Figur 6 dargestellte Modell für die dielektrische Barriereentladung. Die Betrachtung ermöglicht die Ermittlung der Durchbruchspannung (= Zündspannung), die zur Bildung eines Plasmas führt. Unterhalb der Durchbruchspannung liegen Plasmafilamente P vor, die charakteristisch für ein kaltes Plasma beziehungsweise Niederdruckplasma sind.

Ausgangspunkt ist ein Kondensator mit einem Plattenabstand von d=1mm. Zwischen seinen Platten befinden sich Luft. Es sei $\alpha$ die Wahrscheinlichkeit pro Längeneinheit, dass ein Elektron ein neutrales Atom bzw. Molekül ionisiert, wobei Stöße von Ionen mit Neutralatomen aufgrund des schnell wechselnden Feldes und der großen Masse der Ionen vernachlässigt werden können.

Wenn N die Anzahl der entstandenen Elektronen ist, dann gilt:

**6**

$$dN/dx = \alpha N \quad (1.1)$$

$$\Rightarrow N(d) = N_0 e^{\alpha d} \quad (1.2)$$

Dabei ist $N_0$ die Anzahl extern erzeugter Elektronen, beispielsweise durch kosmische Strahlung. Die Zahl ionisierender Stöße ist proportional zum Druck p und zur Wahrscheinlichkeit für einen Ionisationsstoß.

Außerdem gilt für die kinetische Energie der Elektronen

$$E_{ion} = eE\,\lambda_{ion} \quad (1.3)$$

Dabei ist $\lambda_{ion}$ die Beschleunigungsstrecke und E die angelegte elektrische Feldstärke. Aufgrund inelastischer Stöße durchläuft nur ein Bruchteil $\exp(\frac{\lambda_{ion}}{\lambda_{inel}})$ die Strecke $\lambda$ion ohne Energieverlust.

Es folgt für die Konstante $\alpha$ $\quad \alpha = Ap\,e^{-(\frac{\lambda_{ion}}{\lambda_{inel}})} = Ap\exp{-(B\frac{p}{E})} \quad (1.4)$   (1.4)

Mit der Durchbruchspannung $U_{zünd} = E_d$ ergibt sich]

$$U_{zünd} = \frac{Bpd}{\ln(Apd) - \ln(\ln(1+\gamma^{-1}))} \approx 3kV \quad (1.5)$$

Dabei ist $\gamma$ die Zahl der erzeugten Elektronen pro Ion (dritter Townsend-Koeffizient), mit der die Zündbedingung

$$\gamma\cdot(e^{\alpha d}-1) \geq 1 \quad (1.6)$$

lautet. Dabei gilt in der Regel y<<1 Paschenkurve für Luft (Kurve 1) und SF6 (Kurve 2).

   p: Druck,
   s: Spaltgröße.

Die Paschenkurve beschreibt die Abhängigkeit der Durchbruchspannung für die Erzeugung einer Gasentladung vom Produkt aus Spaltgröße und Druck.
Für den vorliegenden Fall kann die Abhängigkeit der Durchbruchspannung von der Spaltbreiten abgeschätzt werden.

| Spaltbreite | $U_{zünd}$ |
|-------------|------------|
| 1 mm | 3 kV |

(fortgesetzt)

| Spaltbreite | $U_{zünd}$ |
|---|---|
| 2 mm | 6 kV |
| 3 mm | 9 kV |
| 4 mm | 12 kV |
| 5 mm | 15 kV |
| 6 mm | 18 kV |

Bei einer Spannung von 3 kV setzt also für Luft bei 1 bar der elektrische Durchbruch ein. Da hier auf der gesamten Strecke d alle Atome bzw. Moleküle ionisiert sind, ist dies die Obergrenze für diejenige Spannung, die für ein stabiles Plasma nötig ist. Unterhalb dieser Spannung bilden sich in einer Barriereentladung dünne Entladungskanäle (Plasmafilamente P) zwischen den Elektroden (Abstand im Bereich 1 mm) aus, die charakteristisch für ein kaltes Plasma sind. Bei Atmosphärendruck wird statistisch verteilt eine hohe Zahl von kurzlebigen Entladungskanälen (Mikroentladungen) beobachtet.

Ein notwendiges Kriterium für die Existenz eines Plasmas ist, dass die Debye-Länge klein gegenüber den Abmessungen des Systems ist. Diese Abschirmlänge ist dadurch charakterisiert, dass auf dieser das Potential einer lokalen Ionen- oder Elektronenladung hinreichend stark (in der Regel auf das 1/e-fache) abgefallen ist. Dies rührt daher, dass in einem Plasma ein positives Ion von einer kugelförmigen Wolke aus Elektronen umgeben ist, so dass sich die Ladungen in etwa kompensieren, wobei der Radius dieser Kugel die Debye-Länge ist. Im vorliegenden Fall ist die Bewegung der Ionen im Wechselfeld gegenüber derjenigen der Elektronen aufgrund der viel größeren Ionenmasse zu vernachlässigen. Deshalb gilt für die Debye-Länge:

$$\lambda_d = \sqrt{\frac{\epsilon_0 k_b T_e}{n_e e^2}} \quad (2.1)$$

Für ein nicht-isothermes Plasma, wobei die Elektronen aufgrund ihrer kleineren Masse eine höhere Temperatur haben als die Ionen, bei einer Barriereentladung

$T_e \sim 1\text{-}10\,eV$ (2.2) (Elektronentemperatur) und

$n_e \sim 10^{20}\text{-}10^{21} m^{-3}$ (2.3) (Volumenanzahldichte der Elektronen).

Setzt man diese Werte in die Gleichung (2.1) ein, so erhält man für die Debye-Länge eines nichtisothermen Plasmas einer Barriereentladung

$$\lambda_d = 2.35 \cdot 10^{-6} m \quad (2.4),$$

wobei diese Debye-Länge für den ungünstigsten Fall einer Anzahldichte von $n_e = 10^{20} m^{-3}$ und einer Elektronentemperatur von $T_e = 10$ eV $= 1{,}16 \cdot 10^5$ K berechnet wurde.

Geht man für den vorliegenden Fall davon aus, dass das System von einer Größenordnung im mm-Bereich ist, dann ist die Debye-Länge um einen Faktor 1000 kleiner, womit das notwendige Kriterium für die Existenz eines Plasmas erfüllt ist.

Ein weiteres Kriterium ist, dass die mittlere Anzahl geladener Teilchen in der Debye-Kugel größer als eins ist. Im ungünstigsten Fall $n_e = 10^{20} m^{-3}$ befinden sich in der Debye-Kugel etwa 5000 geladene Teilchen, womit auch dieses Kriterium erfüllt ist.

Die Parameter der erfindungsgemäßen Vorrichtung erfüllen die physikalischen Voraussetzungen zur Erzeugung eines kalten Plasmas.

| physikalischer Parameter | notwendige Bedingung | plasmaOne | notwendige Bedingung erfüllt ? |
|---|---|---|---|
| Durchbruchspannung | 3 kV bei 1 mm Spalt | 3 bis 18 kV | ja |

(fortgesetzt)

| physikalischer Parameter | notwendige Bedingung | plasmaOne | notwendige Bedingung erfüllt ? |
|---|---|---|---|
| Debye-Länge | Spaltgröße >> $\lambda_d$ = $2.35 \cdot 10^{-6} m$ | Spaltgröße >= 1 mm | ja |
| mittlere Anzahl geladener Teilchen in Debye-Kugel | Anzahl > 1 | Anzahl: ca 5000 | ja |

**Bezugszeichenliste**

1 Transformator
2 Sonde
3 Steuereinrichtung
4 Primärspule
5 Sekundärspule
7 Kupplung
8 Transformatorgehäuse
9 Kupplung
10 Stabkern
11 Kammer
12 Kontaktfeder
13 Energiequelle
14 Metallelektrode
15 Dielektrikum
16 Anregungsspannung
20 Sondenkörper
21 Hohlraum
25 Verbindungsleitung
30 Steckeinrichtung
31 Verbindungskabel
40 Verbindungseinrichtung
50 Verbindung
P Plasmafilemente
B Überlappungsbereich
d1 Abstand
d2 Abstand
F Finger
K Gesamtkapazität
CF Kapazität Finger
L Länge
SK Schwingkreis
G Gewebe

**Patentansprüche**

1. Vorrichtung zur Behandlung von biologischem Gewebe (G) mit einem Niederdruckplasma mit

    a) einem Transformator (1) zur Erzeugung eines hochfrequenten elektromagnetischen Feldes,
    b) einer Sonde (2), welche mit dem Transformator (1) elektrisch koppelbar ist und
    c) einer Steuerungseinrichtung (3) zur Steuerung des durch den Transformator (1) erzeugten hochfrequenten elektromagnetischen Feldes,

**EP 2 934 672 B1**

**dadurch gekennzeichnet, dass**

die Vorrichtung darüber hinaus eine Informationseinrichtung umfasst die der Sonde (2) zugeordnet ist, wobei mit der Informationseinrichtung

die Stromstärke beziehungsweise die Leistung, welche durch die Vorrichtung an das Gewebe (G)abgegeben wird, messbar ist und beim Überschreiten eines vordefinierten Grenzwertes die Energieversorgung der Vorrichtung unterbrechbar ist.

2. Vorrichtung nach Anspruch 1,
   **dadurch gekennzeichnet, dass**
   mit der Informationseinrichtung der Abstand einer Behandlungsfläche der Sonde (2) zu dem zu behandelnden Gewebe (G) messbar sowie optisch und/oder akustisch anzeigbar ist.

3. Vorrichtung nach Anspruch 2,
   **dadurch gekennzeichnet, dass**
   der Transformator (2) ein Transformatorgehäuse (8) umfasst, welches eine einer Kupplung (7) zum Anschluss der Sonde (2) gegenüberliegende Kupplung (9) zum elektrischen/elektronischen Anschluss der Steuereinrichtung (3) aufweist, wobei das Transformatorgehäuse (8) vorzugsweise als Handgriff ausgebildet und entsprechend ergonomisch geformt ist.

4. Vorrichtung nach Anspruch 3,
   **dadurch gekennzeichnet, dass**
   die Steuereinrichtung (3) in dem Transformatorgehäuse (8) angeordnet ist.

5. Vorrichtung nach Anspruch 2 oder 3,
   **dadurch gekennzeichnet, dass**
   die Steuereinrichtung (3) außerhalb des Transformatorgehäuse (8) angeordnet ist.

6. Vorrichtung nach einem der vorhergehenden Ansprüche,
   **dadurch gekennzeichnet, dass**
   an die Steuereinrichtung (3) eine elektrische Energiequelle anschließbar ist.

7. Vorrichtung nach einem der vorhergehenden Ansprüche,
   **dadurch gekennzeichnet, dass**
   die Sonde (2) als Glassonde ausgebildet ist.

8. Vorrichtung nach Anspruch 7,
   **dadurch gekennzeichnet, dass**
   die Glassonde unter Unterdruck, vorzugsweise unter Unterdruck von 500 Pa bis 3000 Pa, besonders bevorzugt von 2000 Pa, mit einem leitenden Gas, vorzugsweise mit einem Edelgas oder Edelgasgemisch gefüllt ist.

9. Vorrichtung nach einem der vorhergehenden Ansprüche,
   **dadurch gekennzeichnet, dass**
   die Sonde (2) mittels einer Kontaktfeder (12) elektrisch/elektronisch mit dem Transformator (1) koppelbar ist, wobei die Kontaktfeder (12) entweder an dem Transformator (1) oder an der Sonde (2) angeordnet ist.

10. Vorrichtung nach Anspruch 1, **dadurch gekennzeichnet, dass** mit der Informationseinrichtung die Zeitdauer der Leistungsabgabe der Vorrichtung an das Gewebe (G) messbar sowie optisch und/oder akustisch anzeigbar ist.

**Claims**

1. Device for treating biological tissue (G) with a low-pressure plasma with

   a) a transformer (1) for generating a high-frequency electromagnetic field,
   b) a probe (2) which can be electrically coupled to the transformer (1) and
   c) a control device (3) for controlling the high-frequency electromagnetic field generated by the transformer (1),

   **characterized in that**

the device comprises an information device associated with the probe (2), whereas the current intensity or the power which is emitted by the device to the tissue (G) is measurable by the information device, and the power supply of the device is interruptible by the information device when a predefined limit value is exceeded.

2.  Device according to claim 1,
    **characterized in that**
    the distance of a treatment surface of the probe (2) to the tissue to be treated (G) is measurable and optically and / or acoustically displayable by the information device.

3.  Device according to claim 2,
    **characterized in that**
    the transformer (1) comprises a transformer housing (8) which has a coupling (9) for electrical / electronic connection of the control device (3) opposite a coupling (7) for connecting the probe (2), wherein the transformer housing (8) is preferably designed as a handle and is ergonomically shaped accordingly.

4.  Device according to claim 3,
    **characterized in that**
    the control device (3) is arranged in the transformer housing (8).

5.  Device according to claim 2 or 3,
    **characterized in that**
    the control device (3) is arranged outside the transformer housing (8).

6.  Device according to one of the preceding claims,
    **characterized in that**
    an electrical energy source can be connected to the control device (3).

7.  Device according to one of the preceding claims,
    **characterized in that**
    the probe (2) is designed as a glass probe.

8.  Device according to claim 7,
    **characterized in that**
    the glass probe is filled with a conductive gas, preferably with a noble gas or noble gas mixture, under depression, preferably under a depression of 500 Pa to 3000 Pa, more preferably of 2000 Pa.

9.  Device according to one of the preceding claims,
    **characterized in that**
    the probe (2) can be coupled electrically / electronically to the transformer (1) by means of a contact spring (12), wherein the contact spring (12) is arranged either at the transformer (1) or at the probe (2).

10. Device according to claim 1,
    **characterized in that**
    the duration of the power output of the device to the tissue (G) is measurable and optically and / or acoustically displayable by the information device.]

**Revendications**

1.  Dispositif pour traiter un tissu (G) biologique avec un plasma basse pression comprenant

    a) un transformateur (1) pour générer un champ électromagnétique à haute fréquence,
    b) une sonde (2) pouvant être couplée électriquement au transformateur (1) et
    c) un dispositif (3) de commande pour contrôler le champ électromagnétique à haute fréquence généré par le transformateur (1),

    **caractérisé en ce que**
    le dispositif comprend un dispositif d'information, qui est associé à la sonde (2), l'intensité du courant ou la puissance émise par le dispositif vers le tissu (G) pouvant être mesurée par le dispositif d'information et l'alimentation de l'appareil pouvant être interrompue lorsqu'une valeur limite prédéfinie est dépassée.

2. Dispositif selon la revendication 1,
   **caractérisé en ce que**
   la distance d'une surface de traitement de la sonde (2) au tissu (G) à traiter est mesurable et affichable optiquement et / ou acoustiquement par le dispositif d'information.

3. Dispositif selon la revendication 2,
   **caractérisé en ce que**
   le transformateur (1) comprend un boîtier (8) de transformateur muni d'un couplage (9) pour le raccordement électrique / électronique du dispositif de commande (3) opposé à un couplage (7) pour connecter la sonde (2), le boîtier de transformateur (8) étant de préférence conçu comme une poignée et ayant une forme ergonomique en conséquence.

4. Dispositif selon la revendication 3,
   **caractérisé en ce que**
   le dispositif (3) de commande est agencé dans le boîtier (8) de transformateur.

5. Dispositif selon la revendication 2 ou 3,
   **caractérisé en ce que**
   le dispositif (3) de commande est agencé à l'extérieur du boîtier (8) de transformateur.

6. Dispositif selon l'une des revendications précédentes,
   **caractérisé en ce qu'**
   une source d'énergie électrique peut être connectée au dispositif (3) de commande.

7. Dispositif selon l'une des revendications précédentes,
   **caractérisé en ce que**
   la sonde (2) est conçue comme une sonde en verre.

8. Dispositif selon la revendication 7,
   **caractérisé en ce que**
   la sonde en verre est remplie d'un gaz conducteur, de préférence d'un gaz noble ou d'un mélange de gaz nobles, sous dépression, de préférence sous une dépression de 500 Pa à 3000 Pa, plus préférablement de 2000 Pa.

9. Dispositif selon l'une des revendications précédentes,
   **caractérisé en ce que**
   la sonde (2) est couplé électriquement / électroniquement au transformateur (1) au moyen d'un ressort (12) de contact, le ressort (12) de contact étant agencé soit au transformateur (1) soit à la sonde (2).

10. Dispositif selon la revendication 1,
    **caractérisé en ce que**
    la durée d'émission de puissance du dispositif au tissu (G) est mesurable et affichable optiquement et / ou acoustiquement par le dispositif d'information.

**Fig. 1**

**Fig. 2**

Fig. 3

Fig. 4a

Fig. 4b

Fig. 4c

Fig. 4d

EP 2 934 672 B1

Fig. 4e

Fig. 4f

Fig. 4g

Fig. 4h

**Fig. 4i**

**Fig. 4k**

**Fig. 4l**

**Fig. 4m**

**Fig. 4n**

**Fig. 4o**

**Fig. 4p**

**Fig. 4q**

Fig. 5

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102005000950 B3
- WO 2006116252 A